# EUROPÄISCHE PATENTSCHRIFT

(11) **EP 1 008 852 B1**
(45) Veröffentlichungstag und Bekanntmachung des Hinweises auf die Patenterteilung: **03.09.2003**
(21) Anmeldenummer: 99122672.1
(22) Anmeldetag: 15.11.1999
(51) Int. Cl.: G01N 33/68

(54) **Verfahren zum spezifischen Nachweis von glykosilierten Proteinen**
Process for the specific detection of glycosylated proteins
Procédé de détection spécifique de protéines glycosylées

(30) Priorität: 08.12.1998 DE 19856433
(43) Veröffentlichungstag der Anmeldung: 14.06.2000
(73) Patentinhaber: Aventis Behring Gesellschaft mit beschränkter Haftung, 35002 Marburg (DE)
(72) Erfinder: Römisch, Jürgen Dr., 35041 Marburg (DE); Feussner, Annette, 35043 Marburg (DE)

(56) Entgegenhaltungen:
- EP-A- 0 337 410
- WO-A-92/08984
- US-A- 4 508 829
- US-A- 5 225 354
- YAGO, HIROKAZU ET AL: "Detection and quantification of soluble asialoglycoprotein receptor in human serum" HEPATOLOGY (PHILADELPHIA) (1995), 21(2), 383-8 , XP000892931
- MORIMOTO K ET AL: "Method for the preparation of bispecific F(ab')2mu fragments from mouse monoclonal antibodies of the immunoglobulin M class and characterization of the fragments" JOURNAL OF IMMUNOLOGICAL METHODS,NL,ELSEVIER SCIENCE PUBLISHERS B.V.,AMSTERDAM, Bd. 224, Nr. 1-2, 22. April 1999 (1999-04-22), Seiten 43-50, XP004165508 ISSN: 0022-1759
- DUARTE, CARLOS A. ET AL: "Epitope mapping, V-region DNA sequence, and neutralizing Fab fragments of two monoclonal antibodies against the HIV-1 V3 loop" IMMUNOTECHNOLOGY (1996), 2(1), 11-20 , XP000891401

## Beschreibung

Gegenstand der Erfindung ist ein Verfahren zur Bestimmung von glykosilierten Proteinen, die an eine Festphase gebunden und nach Umsetzung mit einem markierten Substrat photometrisch nachgewiesen werden.

Die Glykosilierung von Proteinen ist ein komplexer Prozess, der sich im Anschluss an die Proteinsynthese im Zellinneren vollzieht. Durch die Glykosilierung werden Proteine stabilisiert und in einigen Fällen auch erst mit ihren spezifischen Eigenschaften ausgestattet. Den über O- oder N-Glykosilierungen an Aminosäuren geknüpften Zuckern kommen erhebliche physiologische Funktionen zu, zum Beispiel als Teil der von den Zellrezeptoren erkannten Proteinstrukturen, welche wiederum eine Rolle bei Zellaktivierungsprozessen oder der Halbwertszeit von Proteinen spielen können. Obwohl Monosaccharide ubiquitär und nicht für eine bestimmte Spezies charakteristisch sind, finden sich doch nicht selten für bestimmte Lebewesen typische komplexe Zuckerstrukturen, die aus den bekannten Zuckern in unterschiedlicher Weise aufgebaut werden.

Die Glykosilierung spielt eine besondere Rolle bei rekombinant hergestellten Proteinen. Ein beispielsweise in Hefezelien hergestelltes Protein, das der Aminosauresequenz des humanen Proteins völlig gleicht, hat in der Regel ein deutlich unterschiedliches Glykosilierungsmuster, wenn entsprechende Glykosilierungsstellen vorhanden sind. Dies kann zu einer veränderten Halbwertszeit des rekombinanten Proteins, zum Beispiel im Plasma, oder zu einer Induktion von Antikörpern führen, die bei wiederholter Gabe des Proteins allergische Reaktionen provozieren können. Das ist besonders dann von Bedeutung, wenn entsprechend hohe Dosen des Proteins verabreicht werden.

Bei der Expression von rekombinanten Proteinen wird nicht selten beobachtet, dass nur ein Teil des Proteins glykosiliert wird, den es nachzuweisen und ggf. aus dem Produkt zu entfernen gilt. Es ist deshalb wichtig, bereits Spuren von glykosilierten Proteinen nachweisen zu können. Zwar sind einige chemische Methoden zur Bestimmung von glykosilierten Proteinen dem Fachmann vertraut, jedoch sind diese Methoden häufig aufwendig und ermöglichen nicht die gleichzeitige Quantifizierung des spezifischen Proteins in einer komplexeren Lösung, siehe z.B. in WO-A-92/08984. Eine Möglichkeit zum Nachweis von glykosilierten Proteinen bietet auch die ELISA-Technik, wobei spezifische Antikörper gegen die Glykosidstrukturen gerichtet sind und mittels eines zweiten Antikörpers den Nachweis gegen einen anderen Teil der Primärstruktur ermöglichen. Allerdings werden polyklonale und monoklonale Antikörper häufig in Nagetieren wie Kaninchen oder Mäusen hergestellt, die häufig nicht über die Fähigkeit zur Herstellung von Antikörpern verfügen, die gegen Glykosidstrukturen gerichtet sind. In diesen Fällen führt die ELISA-Technik zu keinen brauchbaren Ergebnissen.

Eine Möglichkeit die eine Glykosidstruktur erkennenden Antikörper in einem Nachweisverfahren zu ersetzen, besteht in der Anwendung von Lektinen. Es handelt sich hierbei um spezielle Proteine, die sehr spezifisch Saccharide auch in lipidoder proteingebundener Form erkennen und binden. Sie sind in allen lebenden Organismen weit verbreitet. Die am längsten bekannten Lektine sind ConcanavalinA, Abrin, Ricin und Phasin. Je nach ihrer Art können sie unterschiedliche Glykosidreste binden. Es war deshalb zu vermuten, dass die Bindung glykosilierter Proteine an immobilisiertes Lektin und ihre anschließende Detektion mittels eines spezifischen, markierten Antikörpers gegen die nicht-glykosilierte Region möglich sein sollte. Ebenso war zu erwarten, dass ein Antikörper gegen das spezifische Protein an der Festphase immobilisiert und die Detektion der glykosidischen Strukturen mit einem markierten Lektin durchführbar sein sollte. Es hat sich jedoch gezeigt, dass die Herstellung einer Standardkurve mit entsprechenden Testproteinen zu keiner klaren Abhängigkeit von Konzentration und Messignal führen. Überraschenderweise gelingt dies jedoch, wenn anstelle des vollständigen, monoklonalen Antikörpers (mAb) ein entsprechendes markiertes F(ab)-Fragment verwendet wird.

Gegenstand der Erfindung ist deshalb ein Verfahren zum spezifischen Nachweis von glykosilierten Proteinen, bei dem man
a) die zu untersuchende Probe mit einer Festphase inkubiert, die mit einem Substrat beschichtet ist, welche die an das Protein gebundenen glykosidischen Gruppen immobilisiert,
b) die an die Festphase nicht gebundenen Reste der Probe durch Auswaschen entfernt,
c) auf das immobilisierte, glykosilierte Protein ein enzym-markiertes F(ab)-Fragment oder F(ab')₂-Fragment eines gegen das spezifische Protein gerichteten monoklonalen Antikörper einwirken läßt, und
d) nach Auswaschen des nicht gebundenen, markierten F(ab)-Fragments oder F(ab')₂-Fragments durch Zugabe einer chromogenen oder einer Chemilumineszenz auslösenden Substanz das gebundene, glykosilierte Protein photometrisch nachweist.

Als Substrat zur Beschichtung der Festphase kann ein Lektin eingesetzt werden. Darüber hinaus ist es auch möglich, zur Beschichtung der Festphase einen gegen das spezifische Protein gerichteten, monoklonalen Antikörper oder ein Fragment eines derartigen Antikörpers einzusetzen. Der Nachweis des gebundenen, glykosilierten Proteins kann dann mit einem markierten Lektin durchgeführt werden.

Die überraschende Beobachtung, dass der vollständige, monoklonale Antikörper (mAb) für das erfindungsgemäße Nachweisverfahren nicht einsetzbar ist, wohl aber das entsprechende markierte F(ab)-Fragment, insbesondere das F(ab')₂-Fragment verwendet werden kann, wurde durch die Beobachtung erklärbar, dass der vollständige monoklonale Antikörper zu über 90% an eine ConA-Matrix (ConcanavalinA-Matrix) gebunden wird, während über 99% des F(ab')₂-Fragments im Säulendurchlauf zu finden war. Der Grund für diese Beobachtung könnte darin zu sehen sein, dass der vollständige monoklonale Antikörper über glykosilierte Fc-Anteile verfügt, die an der ConA-Matrix gebunden werden, so dass der monoklonale Antikörper dann nicht mehr in ausreichendem Maße für die Detektion des immobilisierten, glykosilierten Proteins zur Verfügung steht.

Zur Bindung der glykosilierten Proteine an die Festphase können also die dem Fachmann bekannten, vorstehend genannten Lektine oder ihren Derivaten verwendet werden. Dabei ist besonders darauf zu achten, dass zweiwertige Kationen anwesend sind, vor allem Magnesium-, Kalzium- und/oder Manganionen in Form ihrer wasserlöslichen Salze, zum Beispiel als Chloride. Die Anwesenheit eines oder mehrer dieser Ionen ist notwendig, um die komplexe Lektinstruktur aufrecht zu erhalten, die für die Bindung der Glykosidgruppen erforderlich ist. Die Verwendung von Lektinen als Mittel zur Immobilisierung von glykosilierten Proteinen kann beispielsweise mit Mikrotiterplatten durchgeführt werden, die mit ConcanavalinA beschichtet sind. Auf die Anwesenheit von zweiwertigen Kationen zur Bindung des glykosilierten Proteins durch das Lektin ist dabei zu achten. Dies gilt auch für die anschließenden Waschvorgänge, die Inkubation mit der zu untersuchenden Probe und die anschließende Nachweisreaktion. Die Anwesenheit der genannten zweiwertigen Kationen ist auch dann wichtig, wenn das markierte Lektin als Detektor in einem entsprechenden Test verwendet wird.

Ein bevorzugtes Nachweisverfahren für glykosilierte Proteine wird also in der Weise durchgeführt, dass die ein glykosiliertes Protein enthaltende Probe zunächst mit einem an eine Festphase adsorbierten oder gebundenen Lektin inkubiert wird; danach wird die Festphase gewaschen und mit einem Antigen-bindenden Fragment eines für das Protein spezifischen Antikörpers inkubiert, bevorzugterweise einem F(ab')₂-Fragment, das zum Beispiel mit einem Enzym markiert sind. Besonders bewährt hat sich die Detektion mit Hilfe der Peroxidase. Außerdem ist aber auch der Nachweis mittels eines markierten Antigen-bindenden Fragmentes eines Zweitantikörpers möglich. Umgekehrt kann das F(ab')₂-Fragment auch auf der Festphase immobilisiert werden, wobei dann nach Inkubation mit der zu untersuchenden Probe die glykosidischen Gruppen des glykosilierten Proteins mit einem markierten Lektin als Detektorreagenz nachgewiesen werden können.

Die Erfindung wird durch das folgende Beispiel erläutert:

### Beispiel 1:

In diesem Beispiel wurde Albumin verwendet. Dieses enthielt zu einem bestimmten Anteil glykosilierte Moleküle, die es galt nachzuweisen und zu quantifizieren.

Um eine Referenzsubstanz zu gewinnen, wurde glykosiliertes Albumin präparativ mittels einer ConA-Sepharose® (Pharmacia AB, Schweden) gewonnen und durch herkömmliche Proteinbestimmungsmethoden quantifiziert. Aufgrund der Entfernung der verzuckerten Moleküle aus der Albumin-Ausgangslösung und der obengenannten Quantifizierung ließ sich auch der Anteil verzuckerten Albumins in der Ausgangslösung errechnen. Beide Lösungen, also Albumin mit einem bekannten Anteil an glykosiliertem Albumin und das "reine" verzuckerte Albumin dienten zur Erstellung von Standardkurven in dem beschriebenen Testsystem.

Mikrotiterplatten wurden je Vertiefung mit 150 µl einer 10 µg/ml enthaltenden ConcanavalinA (Typ IV-S, Sigma, Deutschland) für 16 Stunden bei Raumtemperatur inkubiert. Der Beschichtungspuffer enthielt 20 mM Na-Acetat, 4 mM MgCl₂, 4 mM CaCl₂, 4 mM MnCl₂, pH 5,5. Nach Waschen der Platten wurden die verschiedenen Verdünnungen des Standards bzw. die Proben appliziert. Diese waren zuvor mit Probenpuffer, bestehend aus 50 mM Tris, 150 mM NaCI, 10 mM MgCl₂, 0,5% Tween 20, pH 7,2, verdünnt worden. Nach einstündiger Inkubation bei 37°C wurden die Vertiefungen dreimal mit Probenpuffer gewaschen und anschließend mit je 100 µl eines mit Peroxidase markierten monoklonalen Antikörpers gegen Human-Albumin bzw. dessen F(ab')₂-Fragmente eine weitere Stunde bei 37°C inkubiert. Nach dreimaligem Waschen wurde ein POD-Substrat in die Vertiefungen pipettiert und für 5 Minuten bei Raumtemperatur (im Dunkeln) inkubiert, die Reaktion gestoppt und die OD₄₅₀ photometrisch bestimmt.

Proben, die eine bekannte Menge an glykosiliertem Albumin enthielten, wurden jeweils mitgeführt und quantifiziert.

### Ergebnis:

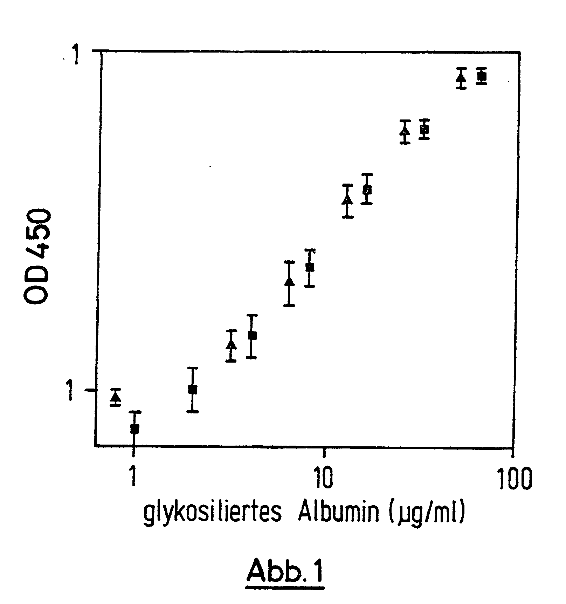

Die Standardkurven wurden an vier aufeinanderfolgenden Tagen erhoben. Die mittels des vollständigen mAb's erhaltenen Ergebnisse waren schlecht reproduzierbar und offenbarten zum Teil keine oder unbefriedigende Abhängigkeiten des Messignals von der eingesetzten Konzentration (nicht gezeigt). Die Mittelwerte und Standardabweichungen der Messwerte hingegen, die mit Hilfe des markierten F(ab')₂-Fragments erhalten wurden, sind in Abbildung 1 gezeigt. Dabei bedeuten:
- Dreiecke reines glykosiliertes Albumin
- Quadrate Albumin mit entsprechendem Anteil an glykosilierten Albumin.

Der parallele und nahezu identische Verlauf bei der Standardkurve zeigt, dass die Reaktion bzw. die Messignale spezifisch sind und nur das verzuckerte Albumin angezeigt wurde.

Die erhaltenen Messwerte mit bekannten Gesamtgehalten an glykosilierten Albumin sind in der folgenden Tabelle dargestellt:

| Probe Nr. | Gehalt an glykosilierten Albumin (%) | gemessener Gehalt an glykosilierten Albumin (%) |
|---|---|---|
| 1 | 0,66 | 0,61 |
| 2 | 0,55 | 0,59 |
| 3 | 0,72 | 0,73 |
| 4 | 0,59 | 0,54 |
| 5 | 0,60 | 0,68 |

## Patentansprüche

1. Verfahren zum spezifischen Nachweis von glykosilierten Proteinen, **dadurch gekennzeichnet, dass** man
a) die zu untersuchende Probe mit einer Festphase inkubiert, die mit einem Substrat beschichtet ist, welche die an das Protein gebundenen glykosidischen Gruppen immobilisiert,
b) die an die Festphase nicht gebundenen Reste der Probe durch Auswaschen entfernt,
c) auf das immobilisierte, glykosilierte Protein ein markiertes F(ab)-Fragment oder F(ab)'₂-Fragment eines gegen das spezifische Protein gerichteten monoklonalen Antikörper einwirken läßt, und
d) nach Auswaschen des nicht gebundenen F(ab)-Fragments oder F(ab)'₂-Fragments den gebundenen Anteil nachweist.

2. Verfahren nach Anspruch 1, **dadurch gekennzeichnet, dass** als Substrat zur Beschichtung der Festphase ein Lektin eingesetzt wird.

3. Verfahren zum spezifischen Nachweis von glykosilierten Proteinen, **dadurch gekennzeichnet, dass** als Substrat zur Beschichtung der Festphase ein gegen das spezifische Protein oder ein gegen die spezifischen Glykosidstrukten gerichtetes F(ab)- oder F(ab)'₂-Fragment eines monoklonalen Antikörpers eingesetzt wird, die an die Festphase nicht gebundenen Anteile ausgewaschen und die gebundenen Anteile nachgewiesen werden.

4. Verfahren nach Anspruch 3, **dadurch gekennzeichnet, dass** zum Nachweis des gebundenen, glykosilierten Proteins ein markiertes Lektin eingesetzt wird.

5. Verfahren nach den Ansprüchen 1 bis 4, **dadurch gekennzeichnet, dass** die Bindung des glykosilierten Proteins an das markierte oder nichtmarkierte Lektin in Gegenwart von zweiwertigen Kationen durchgeführt wird.

6. Assay der das Nachweisverfahrens der Ansprüche 1 bis 5 verwendet.

## Claims

1. A process for the specific detection of glycosylated proteins, which comprises
a) incubating the sample to be investigated with a solid phase which is coated with a substrate which immobilizes the glycosidic groups bonded to the protein,
b) removing the remains of the sample not bound to the solid phase by washing,
c) allowing a labeled F(ab) fragment or F(ab')₂ fragment of a monoclonal antibody directed against the specific protein to act on the immobilized, glycosylated protein, and
d) after washing out the unbound F(ab) fragment or F(ab')₂ fragment, detecting the bound fraction.

2. The process as claimed in claim 1, wherein the substrate employed for coating the solid phase is a lectin.

3. A process for the specific detection of glycosylated proteins, which comprises employing an F(ab) or F(ab')₂ fragment of a monoclonal antibody directed against the specific protein or against the specific glycoside structures, washing out the fractions not bound to the solid phase and detecting the bound fractions.

4. The process as claimed in claim 3, wherein a labeled lectin is employed for the detection of the bound, glycosylated protein.

5. The process as claimed in claims 1 to 4, wherein the binding of the glycosylated protein to the labeled or unlabeled lectin is carried out in the presence of divalent cations.

6. An assay which utilizes the detection process of claims 1 to 5.

## Revendications

1. Procédé de détection spécifique de protéines glycosylées, **caractérisé en ce que**
a) on incube l'échantillon à étudier avec une phase solide qui est recouverte d'un substrat qui immobilise les groupes glycosidiques liés à la protéine,
b) on élimine le résidu d'échantillon non lié à la phase solide par lavage,
c) on laisse agir sur la protéine immobilisée glycosylée un fragment F(ab) ou F(ab)'₂ d'un anticorps monoclonal dirigé contre la protéine spécifique et
d) après lavage du fragment F(ab) ou F(ab)'₂ non liés, on détecte la part liée.

2. Procédé selon la revendication 1 **caractérisé en ce qu'**on utilise une lectine à titre de substrat pour recouvrir la phase solide.

3. Procédé pour la détection spécifique de protéines glycosylées **caractérisé en ce qu'**on utilise un fragment F(ab) ou F(ab)'₂ d'un anticorps monoclonal dirigé contre la protéine spécifique ou contre la structure glycosydique spécifique à titre de substrat pour recouvrir la phase solide, et **en ce que** les parts non liées à la phase solide sont lavées et que les parts liées sont détectées.

4. Procédé selon la revendication 3 **caractérisé en ce qu'**on utilise une lectine marquéee pour la détection de la protéine liée glycosylée.

5. Procédé selon les revendications 1 à 4 **caractérisé en ce que** la liaison de la protéine glycosylée à la lectine marquée ou non marquée est réalisée en présence de cations bivalents.

6. Test qui utilise le procédé de détection des revendications 1 à 5.
